# EUROPEAN PATENT APPLICATION

(11) **EP 0 566 824 A1**
(43) Date of publication of application: **27.10.1993**
(21) Application number: 93100754.6
(22) Date of filing: 20.01.1993
(51) Int. Cl.: C12P 21/00, C07K 1/00

(54) **Enzymatic peptide synthesis**

(30) Priority: 28.01.1992 US 827686
(71) Applicant: F. HOFFMANN-LA ROCHE AG, CH-4002 Basel (CH)
(72) Inventor: Felix, Arthur Martin, West Caldwell, N.J. 07006 (US); Heimer, Edgar Philip, Nutley, N.J. 07110 (US)
(74) Representative: Mezger, Wolfgang, Dr.

(57) **Abstract**

This invention relates to a process for coupling an N-terminal amino acid residue or an N-terminal peptide fragment, each of which can be optionally substituted, to a C-terminal amino acid residue or a C-terminal peptide fragment, each of which can be optionally substituted, to form a compound having the formula

Y_{N}-Q-X_{C} I

where Y_{N} is H or is an N-terminal amino acid residue or an N-terminal peptide fragment, each of which can be optionally substituted, Q is an acidic amino acid residue and X_{C} is a C-terminal amino acid residue or a C-terminal peptide fragment, each of which can be optionally substituted, comprising:
reacting a substrate component having the formula

Y_{N}-Q-OR II

where R is hydrogen, alkyl, haloalkyl, aryl, aralkyl, or a 5- or 6-membered heterocyclic ring having at least one heteroatom,
with X_{C} in the presence of an endopeptidase in an aqueous solution or dispersion having a pH of from about 5 to about 10.5, to form the compound of formula I.

SEQUENCE LISTING

(1) GENERAL INFORMATION:

(i) APPLICANT:
(A) ADDRESSEE: F. Hoffmann-La Roche AG
(B) STREET: Grenzacherstrasse 124
(C) CITY: Basel
(D) STATE: Basel City
(E) COUNTRY: Switzerland
(F) ZIP: CH-4002
(G) Telefon: 061 688 3943
(H) Telefax: 061 688 1395

(ii) TITLE OF INVENTION: ENZYMATIC PEPTIDE SYNTHESIS

(iii) NUMBER OF SEQUENCES: 5

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(2) INFORMATION FOR SEQ ID NO:1:

(i) SEQUENCE CHARACTERISTICS:
(A) LENGTH: 44 amino acids
(B) TYPE: amino acid
(D) TOPOLOGY: linear

(ii) MOLECULE TYPE: peptide

(xi) SEQUENCE DESCRIPTION: SEQ ID NO:1:

Tyr Ala Asp Ala Ile Phe Thr Asn Ser Tyr Ala Lys Val Leu Gly Gln
1               5                   10                  15
Leu Ser Ala Arg Lys Leu Leu Gln Asp Ile Met Ser Arg Gln Gln Gly
            20                  25                  30
Glu Ser Asn Gln Gly Arg Gly Ala Arg Ala Arg Leu
        35                  40

(2) INFORMATION FOR SEQ ID NO:2:

(i) SEQUENCE CHARACTERISTICS:
(A) LENGTH: 26 amino acids
(B) TYPE: amino acid
(D) TOPOLOGY: linear

(ii) MOLECULE TYPE: peptide

(xi) SEQUENCE DESCRIPTION: SEQ ID NO:2:

Ala Ile Phe Thr Asn Ser Tyr Arg Lys Val Leu Ala Gln Leu Ser Ala
1               5                   10                  15
Arg Lys Leu Leu Gln Asp Ile Met Ser Ala
            20                  25

(2) INFORMATION FOR SEQ ID NO:3:

(i) SEQUENCE CHARACTERISTICS:
(A) LENGTH: 26 amino acids
(B) TYPE: amino acid
(D) TOPOLOGY: linear

(ii) MOLECULE TYPE: peptide

(xi) SEQUENCE DESCRIPTION: SEQ ID NO:3:

Ala Ile Phe Thr Asn Ser Tyr Arg Lys Val Leu Ala Gln Leu Ser Ala
1               5                   10                  15
Arg Lys Leu Leu Gln Asp Ile Met Ser Arg
            20                  25

(2) INFORMATION FOR SEQ ID NO:4:

(i) SEQUENCE CHARACTERISTICS:
(A) LENGTH: 29 amino acids
(B) TYPE: amino acid
(D) TOPOLOGY: linear

(ii) MOLECULE TYPE: peptide

(xi) SEQUENCE DESCRIPTION: SEQ ID NO:4:

Xaa-Xaa Asp Ala Ile Phe Thr Asn Ser Tyr Arg Lys Val Leu Ala Gln
1 5 10 15
Leu Ser Ala Arg Lys Leu Leu Gln Asp Ile Met Ser Arg
20 25

(2) INFORMATION FOR SEQ ID NO:5:

(i) SEQUENCE CHARACTERISTICS:
(A) LENGTH: 29 amino acids
(B) TYPE: amino acid
(D) TOPOLOGY: linear

(ii) MOLECULE TYPE: peptide

(xi) SEQUENCE DESCRIPTION: SEQ ID NO:5:

Xaa Xaa Asp Ala Ile Phe Thr Asn Ser Tyr Arg Lys Val Leu Ala Gln
1               5                   10                  15
Leu Ser Ala Arg Lys Leu Leu Gln Asp Ile Met Ser Ala
            20                  25

## Description

This invention relates to peptide synthesis, and in particular, to the enzymatic coupling of two or more peptide fragments and/or amino acid residues.

It is known that syntheses of peptides may be carried out by more or less sophisticated coupling reactions, both in respect of homogeneous synthesis and of heterogeneous solid phase synthesis. All these chemical methods, however, involve the risk of undesirable secondary reactions and racemizations, and it is therefore necessary to control the chemical reactions carefully to minimize or eliminate these problems. Moreover, the amino acid side chains must often be protected, requiring deblocking as the last chemical step to produce the desired peptides. Depending upon the size of the synthesized peptide, the yields may be low, and the secondary reactions frequently necessitate cumbersome purification procedures to obtain the pure peptide. All these inherent problems of chemical peptide syntheses plus the high price of several of the coupling reagents and the blocking amino acid derivatives mean that even small synthetic peptides, e.g. pentapeptides, are relatively expensive to produce.

There is interest in the use of proteolytic enzymes to catalyze formation of peptide bonds in the synthesis of bioactive compounds, such as peptides (oligo- and polypeptides) (Fruton, J.S. (1982) Adv. Enzymol. Relat. Areas Mol. Biol. 53, 239-306; Nakanishi, K. & Matsuno, R. (1988) Adv. Biotechnol. Processes 10, 173-202). The use of these catalysts is attractive for use in peptide synthesis, and organic synthesis in general, because of their high regioselectivity and stereoselectivity.

Enzymatic coupling can be applied to a fragment-condensation strategy for the synthesis of peptides. Fragment condensations are often hindered by low solubility of one or both of the side-chain protected peptide fragments. The high regioselectivity of the proteases permits enzymatic coupling without the need for side-chain protection. Fragment condensations via conventional stoichiometric coupling reagents can also be plagued by considerable racemization at the α-carbon atom of the carboxyl donor. The stereospecificity of the proteases ensures products of highest optical purity. A kinetically-controlled method of enzymatic coupling is particularly promising with regard to fragment condensation.

Enzymatic peptide synthesis can also be used for semisynthesis of peptide analogs from biosynthetic precursors. Potentially useful semisynthetic modifications of biosynthetic peptides include incorporation of "unnatural" amino acid side-chains, C-terminal amidation, and other structural features including the fragments containing non-naturally occuring amino acids that are not available from existing recombinant DNA methods.

One area of specific interest involves producing peptides with growth hormone releasing activity which can be used to treat a variety of growth hormone related problems in humans and for performance enhancement in animals and aquaculture. The background of use of growth hormone releasing (GRF) factors are set forth e.g. in United States Patent Nos. 4,622,312; 4,649,131; 4,732,972; and 4,734,399. However, other types of peptides and compounds can also be made within the scope of this invention.

Thus, an object of this invention is to couple two amino acid residues and/or peptide residues using an endopeptidase which cleaves at the carboxyl end of an acidic amino acid residue. An example of such an endopeptidase is V8 protease from S. aureus. This invention is well suited to coupling peptide fragments or residues which are produced by traditional peptide synthesis, such as solid phase peptide synthesis, or produced by recombinant DNA synthesis.

Therefore, in its broaded sense this invention relates to a process for coupling an N-terminal amino acid residue or an N-terminal peptide fragment, each of which can be optionally substituted, to a C-terminal amino acid residue or a C-terminal peptide fragment, each of which can be optionally substituted, to form a compound having the formula

Y_{N}-Q-X_{C} I

where Y_{N} is H or is an N-terminal amino acid residue or an N-terminal peptide fragment, each of which can be optionally substituted, Q is an acidic amino acid residue and X_{C} is a C-terminal amino acid residue or a C-terminal peptide fragment, each of which can be optionally substituted, comprising:
reacting a substrate component having the formula

Y_{N}-Q-OR II

where R is hydrogen, alkyl, haloalkyl, aryl, aralkyl, or a 5- or 6-membered heterocyclic ring having at least one heteroatom,
with an amino acid or peptide X_{C} in the presence of an endopeptidase in an aqueous solution or dispersion having a pH of from about 5 to about 10.5, to form the compound of formula I.

The endopeptidase can be of yeast, animal, vegetable, or microbial origin.

Endopeptidase refers to an enzyme which internally cleaves a peptide. Preferably, the endopeptidase cleaves specifically at the carboxyl side of an acidic amino acid residue. Acidic amino acids are e.g. glutamic acid or aspartic acid. An example for such an endopeptidase is the V8 protease from S. aureus.

In a preferred embodiment, the invention relates to a process wherein a substrate component II having the formula

desNH₂-Tyr-D-Ala-Asp(OH)-OEt

is reacted with a compound X_{C} of formula 
Ala Ile Phe Thr Asn Ser Tyr Arg Lys Val Leu Ala Gln Leu Ser Ala
1               5                   10                  15
Arg Lys Leu Leu Gln Asp Ile Met Ser Arg-NH2
20 25
(= SEQ ID No: 3-NH₂) to form a product of formula I having the sequence 
desNH2-Tyr D-Ala Asp Ala Ile Phe Thr Asn Ser Tyr Arg Lys Val Leu
5 10
Ala Gln Leu Ser Ala Arg Lys Leu Leu Gln Asp Ile Met Ser Arg-NH2
15 20 25
Examples of useful amino acids for the present invention are aliphatic amino acids, such as monoamino monocarboxylic acids, e.g. glycine (Gly), alanine (Ala), valine (Val), norvaline (Nva), leucine (Leu), isoleucine (Ile), and norleucine (Nle), hydroxy amino acids, such as serine (Ser), threonine (Thr) and homoserine (homo-Ser), sulfur-containing amino acids, such as methionine (Met) or cystine (CysS) and cysteine (CysH), monoamino dicarboxylic acids, such as aspartic acid (Asp), glutamic acid (Glu), asparagine (Asn) and glutamine (Gln), diaminomonocarboxylic acids, such as ornithine (Orn), lysine (Lys) and arginine (Arg), aromatic amino acids, such as phenylalanine (Phe) and tyrosine (Tyr), as well as heterocyclic amino acids, such as histidine (His) and tryptophan (Trp).

The invention also relates to the peptides or polypeptides produced by the process as described above.

As protective groups may be used the amino protective groups common within the peptide chemistry, such as benzyl (Bzl-), acetyl (Ac-) or tertiary alkoxycarbonyl groups, e.g. t-butyloxycarbonyl (Boc-), t-amyloxycarbonyl (t-AOC-), benzyloxycarbonyl (Z-), p-methoxybenzyloxycarbonyl (PMZ-), 3,5-dimethoxy-benzyloxycarbonyl (Z(OMe)₂-), 2,4,6-trimethyl-benzyloxycarbonyl (TMZ-), p-phenylazobenzyloxycarbonyl (PZ-), p-toluenesulfonyl (Tos-), o-nitrophenylsulfenyl (Nps-), dichlorobenzyl (Dcb-), 2-chlorobenzyloxycarbonyl (2CIZ-), cyclohexylester (OcHex-), fluorenyl-9-ylmethyloxycarbonyl (Fmoc-) or the like.

Preferred protective groups are benzyloxycarbonyl and t-butyloxycarbonyl since these derivatives are easy to produce, economically attractive and easy to remove.

As used herein, "alkyl" means straight chain or branched alkyl, preferably with 1 to 6 carbon atoms, e.g. methyl, ethyl, propyl, isopropyl, butyl, isobutyl, tert. butyl, amyl, hexyl and the like.

As used herein, "haloalkyl" means alkyl substituted by a halogen such as fluorine, bromine, chlorine, and iodine.

As used herein, "aralkyl" means a hydrocarbon group having both aromatic and aliphatic structures; that is, a substituted or unsubstituted aryl group is substituted for a hydrogen atom of an alkyl group. Examples include benzyl, phenethyl, α-mesityl, etc.

All of these groups may be substituted with substituents which are inert with relation to the enzyme, e.g. halo (fluoro, chloro, bromo, iodo), nitro, alkoxy (methoxy, ethoxy, etc.), or alkyl (methyl, ethyl, etc.), provided the amino acid residue or the peptide derivative is a substrate for the endopeptidase.

As used herein, "aryl" means a substituted or unsubstituted aromatic moiety derived from an aromatic hydrocarbon by the removal of one hydrogen atom such as phenyl, tolyl, xylyl, mesityl, cumenyl, naphthyl and the like wherein the aryl group may have 1 to 3 suitable substituents such as halo (fluoro, chloro, bromo, iodo), hydroxy, amino, nitro, and the like. The aromatic hydrocarbon from which aryl is derived can be mononuclear, binuclear or polynuclear aromatic hydrocarbon.

As used herein, "heterocyclic ring" refers to an unsubstituted or substituted, saturated, unsaturated, or aromatic ring radical comprised of carbon atoms and at least one heteroatom in the ring. Heterocyclic rings are monocyclic or polycyclic. Polycyclic ring systems are fused, bridged or spiro. Monocyclic rings contain from 3 to 9 atoms preferably 3 to 7. Polyclic rings contain 7 to 17 atoms, preferably 7 to 13 atoms. Examples of substituents include lower alkyl, lower alkoxy, halogen, halogen substituted-lower alkyl, amino, mercapto, cyano, hydroxy, carbamoyl, carboxy groups, etc. and others. Examples of monocyclic heterocyclic rings include pyrrolidinyl; imidazolidinyl; pyrazolidinyl; pyrrolinyl; pyrrolyl; imidazolyl; furyl; thienyl; 1H-tetrazolyl; 1,2,3-thiadiazolyl; 1,2,4-thiadiazolyl; 1,3,4-triazolyl; 1,2,3-oxadiazolyl; 1,2,4-oxadiazolyl; 1,3,4-oxadiazolyl; 1,2,5-oxadiazolyl; 1,2,4-triazolyl; piperidyl; piperazinyl; pyridyl; pyrazinyl; pyrimidinyl; morpholinyl; 4-thiamorpholinyl; 2-amino-4-thiazolyl; 5-amino-1,2,4-thiadiazolyl; etc., and others. Examples of polycyclic heterocyclic rings include benzofuranyl, 4,1,2-benzoxathiozinyl, 2,1,4-benzoxadiazinyl, 2,1-benzoxazinyl, etc.

As used herein, "heteroatom" refers to nitrogen, oxygen or sulfur.

"Acidic amino acid residue" refers to those amino acids having an acid side chain. Examples include Asp, Glu and the like.

It should be mentioned that ionizable groups which may be present in the individual amino acids, may, if desired, be blocked in a manner known to those of ordinary skill in the art depending upon the type of the group. Suitable protective groups for the ω-amino group (N^{ω}) include e.g. N^{ω}-benzyloxycarbonyl (N^{ω}-Z), t-butoxycarbonyl (N^{ω}-Boc) or tosyl (N^{ω}-Tos). Suitable protective groups for the N-guanidino group (N^{G}) in Arg include, for example, Nitro (N^{G}-NO₂), N^{G}-benzyloxycarbonyl (N^{G}-Z) and N^{G},N^{G}-dibenzyloxycarbonyl (N^{G}-Z-Z). Suitable protective groups for the imidazole ring (N^{im}) in His includes, for example, N^{im}-benzyl (N^{im}-Bzl) and Tosyl (N^{im}-Tos). Suitable protective groups for the ω-carboxyl groups include, for example, ω-benzyl ester (-OBzl). Suitable protective groups for the hydroxyl group in aliphatic or aromatic hydroxy amino acids include, for example, aralkyl groups, such as benzyl (Bzl-). Suitable S-protective groups for the mercapto group in CysH include, e.g. the benzyl group (Bzl-). The protective groups must be stable during the primary reaction and be easy to cleave from the final product without causing any secondary reactions.

Where the amino acid residue has an isomeric form, it is the L-form of the amino acid that is represented unless otherwise expressly indicated. The suffixes "OH" and "NH₂" following "SEQ ID NO:#" (where # is the sequence number) at the C-terminus refers to the free carboxylic acid and amide forms of the compound, respectively. In the event neither suffix is used, the expression is intended to encompass both forms. The term "BOC-SEQ ID NO:# - PAM resin" refers to a protected sequence of SEQ ID NO:# where BOC protects the amino group at the N-terminus and PAM-resin protects the C-terminus. The compounds can encompass the free carboxylic acid form or the amide form. The basic GRF molecule contains 44 amino acids as is shown in SEQ ID NO: 1. Analogs of GRF are indicated by setting forth the substituted amino acid in brackets before "GRF"; for example, "[His¹,Ala¹⁵]-GRF" indicates a polypeptide having an amino acid sequence corresponding to GRF in which a histidine residue has been substituted for the tyrosine residue at position 1 and an alanine residue has been substituted for the glycine residue at position 15. Numbers in parentheses following "GRF" indicate fragments of the full polypeptide by giving the position numbers of the amino acid residues; for example, GRF (1-29) indicates a fragment having the first 29 amino acids of the full sequence.

Prefixes and suffixes other than "OH" and "NH₂" preceding or following "SEQ ID NO:#" refers to a protected sequence of "SEQ ID NO:#" where the protection groups are shown above. When Xaa carries a superscripted member, the number refers to position of Xaa in the particular sequence, starting at the N-terminal end and going towards the C-terminal end.

The following examples are presented to illustrate one aspect of the process of the invention. The examples are not intended in any way to limit the scope of the invention. The examples were carried out as set forth below.

### EXPERIMENTAL PROCEDURE

All amino acid derivatives were purchased from Bachem, Inc. (Torrance, CA). Side-chain protected amino acids used were: Boc-Arg(Tos)-OH, Boc-Asp(OcHex)-OH, Boc-Lys(2ClZ)-OH, Boc-Ser(Bzl)-OH, Boc-Thr(Bzl)-OH, and Boc-Tyr(Dcb)-OH. Solid phase peptide synthesis was performed on the Applied Biosystems 430A Peptide Synthesizer (Foster City, CA). Preparative HPLC was carried out on the Waters Delta Prep 3000 instrument. Analytical HPLC was performed using an LDC Constametric IIG assembly equipped with a gradient master and Spectromonitor III U.V. variable wavelength detector. Carboxypeptidase Y (CPD-Y) was obtained from Carlbiotech Ltd., Copenhagen, Denmark. This enzyme is more fully described in US Patent Nos. 4,339,534 and 4,806,473, both of which are hereby expressly incorporated by reference.
V8 Protease (Protease from the V8 strain of staphylococcus aureus, endoproteinase Glu-C) was obtained from Sigma Chemical Co. (Type XVII-B) and had a specific activity of 770 U mg⁻¹ (1U = amount of enzyme that hydrolyzes 1 µmole min⁻¹ N-t-Boc-Glu-αOPh at pH 7.8, 37°C).

All temperatures are in degrees Celsius.

### Example 1

### Preparation of desNH₂Tyr-D-Ala-Asp(OH)-OEt

A solution of 1.0 g (6.21 mmol) of L-aspartic acid α-ethyl ester in H₂O (10 mL) containing 0.52 g (6.21 mmol, 1 eq) of NaHCO₃ was treated with 1.95 g (6.8 mmol, 1.1 eq) of Boc-D-Ala-OSu in ethylene glycol dimethyl ether. Stirring proceeded at 25° for 1.5 hours and at 4° overnight. The reaction mixture was acidified to pH 3.5 using solid citric acid and evaporated to dryness. The residue was taken up in H₂O (100 mL) and lyophilized. The resultant product, Boc-D-Ala-Asp(OH)-OEt, was treated with 50% TFA-CH₂Cl₂(60 mL) for 20 minutes at 25°. The solution was evaporated to dryness and reevaporated twice from CH₂Cl₂ (60 mL each). The residue was dissolved in H₂0 (50 mL) and solid NaHCO₃ added to pH 7.4. A solution of desNH₂-L-Tyr-OSu (1.95 g, 7.4 mmol, 1.2 eq) in ethylene glycol diethyl ether (100 mL) was added and stirring proceeded at 4° overnight. The reaction mixture was evaporate, taken up in H₂0 (100 mL), acetonitrile added (∼10 mL) followed by 50% aqueous TFA to pH 2.8. Following filtration (0.45A filter), the solution was applied onto a YMC phenyl column (2.0 x 30 cm) and purified by preparative HPLC. Eluant: (A) H₂O (0.025% TFA) - (B) CH₃CN (0.025% TFA) in a linear gradient from 0% (B) to 35% (B) in 90 min; flow rate 15 mL/min.

Fractions were collected at 1 minute intervals. The product emerged between fractions 43-62 which were pooled and evaporated to give 1.25 g (53.1%) of desNH₂Tyr-D-Ala-Asp(OH)-OEt. The product was shown to be homogeneous by analytical HPLC and gave the expected amino acid composition after acid hydrolysis (6N HCl; 110°; 24h): Asp, 1.00; Ala, 0.99. The structure was confirmed by ¹H NMR and compatible with the molecular formula for C₁₈H₂₄N₂O₇ by FAB-MS: Calcd: (M+H)+ 381.4; Found: 381.3

### Example 2

### Synthesis of SEQ ID NO:2-OH

t-Butyloxycarbony-L-alanyl-O-phenylacetamidomethyl-resin (Boc-Ala-PAM-resin) (0.64 g; 0.5 mM) was charged into the reaction vessel of an Applied Biosystems (AB) 430A automatic peptide synthesizer. Solid phase peptide synthesis was carried out using the AB 430A protocol with the coupling times extended to 2 hours to give 2.2g of Boc-SEQ ID NO:2-PAM-resin, {Boc-[Ala¹⁵,Ala²⁹]-GRF(4-29)-PAM-resin}. A 1 g portion of the protected peptide resin was treated with anhydrous HF using the "low-high" procedure [Tam, J.P., Heath, W.F. and Merrifield, R.B. (1983) J. Am. Chem. Soc., 105, 6442-6445] and the crude peptide resin triturated with EtOAc and extracted with TFA. The solvent was evaporated and the residue triturated with ether and dried to give 660 mg of crude SEQ ID NO:2-OH ,{[Ala^{15,29}]-GRF(4-29)-OH}.

The crude material (660 mg) was dissolved in 25 mL of 0.1% TFA/H₂O, filtered (0.45 micron type HA Millipore filter) and loaded onto a YMC ODS Basic Prep-PAK HPLC column. The column was eluted with (A) H₂O (0.1% TFA) - (B) CH₃CN (0.1% TFA) in a linear gradient from 20% (B) to 50% (B) in 90 minutes with a flow rate of 50 mL/min. Fractions were collected (0.5 min/fraction) and aliquots analyzed by the analytical HPLC system: (A) 0.1M NaClO₄ (pH 2.5) - (B) CH₃CN; 40% (B) to 55% (B) in 20 min at 1 mL/min, 0.2 AUFS, 206 nm. Column: Lichrosorb RP-8 (5 microns). The product emerged in fractions 78-85 which were combined, evaporated and lyophilized to give pure SEQ ID NO:2-OH, {[Ala^{15,29}]-GRF(4-29)OH}. Yield: 125 mg.

The product was shown to be homogeneous by analytical HPLC and gave the expected amino acid composition after acid hydrolysis (6N HCl - 1% TGA, 150°C, 1h): Thr 0.97 (1); Ser 3.01(3); Tyr 1.02 (1). 110°C; 24h: Asp 2.01 (2); Glu 2.07 (2); Ala 4.18 (4); Val 0.91 (1); Met 1.03 (1); lle 1.91 (2); Leu 4.07 (4); Phe 0.94 (1); Lys 1.86 (2); Arg 2.00 (2). Confirmation of structure was provided by FAB mass spectrometry. Calcd: (M+H)⁺ 2939.5. Found: 2938.7.

### Example 3

### Enzymatic Synthesis of SEQ ID NO:3-NH₂

Arg-NH₂·2HCl (353.5 mg) was dissolved in water (0.49 mL) followed by the addition of 3.0M NaOH (0.22 mL), 1M Na-EDTA (0.005 mL) carboxypeptidase-Y (0.041 mg in 0.002 mL H₂O) and the solution was heated to 37°C (final pH = 7.70). A portion of this solution (0.350 mL) was added to a solution of SEQ ID NO:2-OH [10.14 mg from Example 2] in H₂O (0.350 mL) at 37°C. The reaction was allowed to proceed at 37°C for 1.1 hr and halted by the addition of glacial acetic acid (2.0 mL). The reaction mixture was loaded onto an Alltech (5µ) mixed mode (cation) HPLC column (1 x 25 cm) and the column was washed (4.0 mL/min) with (A) H₂O (0.1% TFA) - (B) CH₃CN (0.1% TFA) in a stepwise gradient: 10% (B) for 20 min, 15% (B) 20 min. and the product eluted using a linear gradient going from 20% to 34% (B) in 40 min. Fractions were collected (every 0.5 min) and analyzed on the analytical HPLC system (as in Example 2) and the product-containing fractions were pooled and lyophilized to yield 5.30 mg of SEQ ID NO:3-NH₂, {[Ala¹⁵]-GRF(4-29)-NH₂}.

The product was shown to be homogeneous by analytical HPLC and gave the expected amino acid composition after acid hydrolysis (6N HCl - 1% TGA; 110°C; 72h): Asx 1.99 (2); Thr 0.96 (1); Ser 2.69 (3); Glx 1.95 (2); Ala 2.94 (3); Val 0.84 (1); Met 1.00 (1); lle 1.84 (2); Leu 4.11 (4); Tyr 1.02 (1); Phe 0.98 (1); Lys 2.22 (2); Arg 3.46 (3). Further confirmation of structure was provided by FAB mass spectrometry. Calcd: (M+H)⁺ 3023.6. Found: 3023.8

### Example 4

### Enzymatic Synthesis of SEQ ID NO:4-NH₂ where Xaa¹ is desNH₂Tyr and Xaa² is D-Ala Using S. aureus V8 Protease

DesNH₂Tyr-D-Ala-Asp(OH)-OEt tripeptide (2.69 mg from Example 1) was dissolved in 0.5M Na₂HPO₄/H₃PO₄ buffer (31.0 µL, pH 10.0) and 0.33 mg of Staphyllococcus aureus V8 protease was added.

The coupling reaction was initiated by the addition of a solution of SEQ ID NO:3-NH₂ (2.0 mg from Example 3) in dimethylacetamide (7.0 µL) and the reaction mixture left standing at 35°C for 2 hr. At the end of this time the reaction was terminated by the addition of glacial acetic acid (0.050 mL) and the reaction mixture was subjected to HPLC purification as in Example 2 to give 0.5 mg of pure SEQ ID NO:4-NH₂ where Xaa¹ is desNH₂Tyr and Xaa² is D-Ala.

The product was shown to be homogeneous by analytical HPLC and gave the expected amino acid composition after acid hydrolysis (6N HCl; 110°C; 24h): Asx 3.07 (3); Thr 0.90 (1); Ser 2.53 (2); Glx 2.04 (2); Ala 4.40 (4); Val 0.94 (1); Met 1.02 (1); Ile 2.07 (2); Leu 4.31 (4); Tyr 1.01 (1); Phe 1.01 (1); Lys 1.98 (2); Arg 3.08 (3). Further confirmation of structure was provided by FAB mass spectrometry. Calcd: (M+H)⁺ 3357.9. Found: 3358.1.

### Example 5

### Alternative Enzymatic Synthesis of SEQ ID NO: 4-NH₂ Where Xaa¹ is desNH₂Tyr and Xaa² is D-Ala

DesNH₂Tyr-D-Ala-Asp(OH)-OEt (16.1 mg, 42 µmol) was dissolved in 280 µL of water, titrated to pH 8.4 (glass electrode) with 1 M Tris base (45 µL), then diluted with water to a final volume of 645 µL. SEQ ID NO:2-OH, {[Ala^{15,29}]-GRF(4-29)-OH}·5TFA (22.3 mg, 6.4 µmol) was dissolved in 360 µL of DMSO, followed by 630 µL of water, followed by 630 mL of the above desNH₂Tyr-D-Ala-Asp(OH)-OEt solution. The reaction was initiated by adding 180 µL of 4.8 mg mL⁻¹ V8 protease at 37°C and the reaction was maintained at 37°C. The progress of the reaction was monitored by removing 2 µL aliquots and analyzing by HPLC: column: Waters µBondapak C₁₈ (5µ, 0.4 x 30 cm), eluants: (A) 0.025% TFA - (B) CH₃CN(0.025% TFA), gradient: 10 to 42% B in 15 min, flow: 1.5 mL min⁻¹; detection: 206 nm.

The reaction was halted at the 76 min mark (60% conversion by HPLC) by adding glacial acetic acid (0.5 mL). The quenched reaction mixture was fractionated by HPLC: column: Vydac C₁₈ 218TP1022 (2.2 x 25 cm), eluants: (A) 0.1% TFA - (B) CH₃CN(0.1% TFA), gradient: 20 to 40% B in 60 min, flow: 25 mL min⁻¹; detection: 210 nm. Product-containing fractions were pooled and lyophilized to yield 8.0 mg (2.1 µmol, 33%) of pure semisynthetic SEQ ID NO:5-OH where Xaa¹ is desNH₂Tyr and Xaa² is D-Ala {[desNH₂Tyr¹,D-Ala²,Ala^{15,29}]-GRF(1-29)-OH}.

The product was shown to be homogeneous by analytical HPLC and gave the expected amino acid composition after acid hydrolysis (6N HCl - 1% TGA; 110°C; 72 h): Asx 2.9 (3); Thr 1.2 (1); Ser 2.4 (3); Glx 3.0 (3); Ala 4.6 (5); Val 1.2 (1); Met 0.8 (1); Ile 2.0 (2); Leu 4.2 (4); Tyr 0.9 (1); Phe 0.9 (1); Lys 2.1 (2); Arg 2.0 (2). FAB mass spectrometry: Calcd (M+H)⁺ 3273.8; Found: 3273.

### Carboxypeptidase Y-catalyzed transpeptidation

Arg-NH₂·2HCl (0.53 g) was dissolved in 500 µL
of water, 50 µL of 0.1 M EDTA, titrated to pH 8.0 (glass electrode) with 5 M Tris base (750 µL). SEQ ID NO:5-OH where Xaa¹ is desNH₂Tyr and Xaa² is D-Ala, {desNH₂Tyr¹,D-Ala²,Ala^{15,29}]-GRF(1-29)-OH} (7.2 mg, 1.9 µmol) was dissolved in 27 µL of water, followed by 70 µL of the above Arg-NH₂ solution at 37°C. The reaction was initiated by adding 3.0 µL of 1.0 mg mL⁻¹ carboxypeptidase Y and the reaction mixture was maintained at 37°C. The progress of the reaction was monitored by removing 1.5 µL aliquots and analyzing by HPLC: column: Vydac C₁₈ 218TP54 (0.46 X 25 cm), eluants: (A) 0.025% TFA - (B) CH₃CN(0.025% TFA), gradient: 38 to 44% B in 10 min, flow: 1.5 mL min⁻¹; detection: 206 nm.

The reaction was halted at the 62 min mark (70% conversion by HPLC) by adding glacial acetic acid (0.6 mL). The quenched reaction mixture was fractionated by HPLC: column: Vydac C₁₈ 218TP1022 (2.2 x 25 cm), eluants: (A) 0.1% TFA - (B) CH₃CN(0.1% TFA), gradient: 20 to 40% B in 60 min, flow: 25 mL min⁻¹; detection: 210 nm. Product-containing fractions were pooled and lyophilized to yield 3.8 mg (0.97 µmol, 51% yield, 17% overall) of pure semisynthetic SEQ ID NO:4-NH₂ where Xaa¹ is desNH₂Tyr and Xaa² is D-Ala, {[desNH₂Tyr¹,D-Ala²,Ala¹⁵] GRF(1-29)-NH₂}.

The product was shown to be homogeneous by analytical HPLC and gave the expected amino acid composition after acid hydrolysis (6N HCl - 1% TGA; 110°C; 72 h): Asx 3.3 (3); Thr 1.0 (1); Ser 2.5 (3); Glx 2.3 (3); Ala 4.0 (4); Val 1.0 (1); Met 0.9 (1); Ile 2.0 (2); Leu 4.2 (4); Tyr 1.0 (1); Phe 1.0 (1); Lys 2.0 (2); Arg 2.9 (3). FAB mass spectrometry: Calcd (M+H)⁺ 3357.9, Found: 3358. The HPLC of the tryptic digest of the product was identical to that of standard SEQ ID NO:4 where Xaa¹ is desNH₂Tyr and Xaa² is D-Ala produced by solid phase peptide synthesis. See U.S. Patent No. 4,649,131, issued March 10, 1987, at column 14, lines 47-67, which is incorporated herein by reference. *In vitro* bioassay: 4.33 relative potency (compared to GRF (1-44)-NH₂ [SEQ ID NO: 1-NH₂]). For determination of the *in vitro* bioassay, see Example 6 below.

### Example 6

The biological activity of the novel peptides were compared with that of a synthetic standard of the natural sequence of GRF(1-44)-NH₂ (SEQ ID NO: 1-NH₂) which was isolated from a human pancreatic tumor of an individual suffering from acromegaly (Salk Institute standard hp-GRF-NH₂(NL-A-10)). The assay for biological activity, which is based on the ability to stimulate production of growth hormone in rat pituitary cells in tissue culture, was performed in the following manner.

Pituitaries from 30-40 male Sprague-Dawley rats (175 g) were removed aseptically after decapitation. The anterior lobes were collected, washed 3 times in sterile Hepes buffer (0.025M)(pH 7.35) and dispersed at 37°C. in 20-30 ml Hepes buffer (pH 7.35) containing collagenase (4 mg per ml) and Dispase (Protease grande II, 2 mg per ml). After gentle 80 min. vortexing and trituration by Pasteur pipette, the dispersed cells were separated by centrifugation (150 X g, 4 min.) and re-suspended in Hepes buffer containing neuraminidase (4 mg/ml), and 200 mg/ml ethylenediaminetetraacetic acid (EDTA) disodium salt pH 7.35, for 10 min. The cells were washed twice with plating medium and plated on multiwell-plates (1.5x10⁵ cells per ml) using the following defined medium: F-12/DMEM/BGJ(6:3:1) (Gibco: 430-1700/430-1600/320-2591) with 2 g BSA/L., 2.38 g Hepes/L., 50 mg Gentamycin/L (Schering Co.). The medium in each well was supplemented either with the novel peptide or natural GRF(1-44)-NH₂ (SEQ ID NO: 1-NH₂) at concentrations ranging from 3.1 to 200 fmol. per ml. of medium. Control wells contained no supplement. Plating was done with this medium added with 2% fetal calf serum to ensure rapid fixation of the cells. On the fourth day the cells were washed twice with the defined medium without fetal calf serum. Finally 900 ml of defined medium was added to each well plus 100 ml of the same medium containing each individual treatment, in triplicate. After 3 hours of incubation the medium was collected and diluted as required to conduct radioimmunoassay (RIA) for rat growth hormone. RIAs were conducted using Sinha's anti-murine GH immune serum and procedures according to the National Pituitary Agency using protein A to precipitate antibody antigen complex.

## Claims

1. A process for coupling an N-terminal amino acid residue or an N-terminal peptide fragment, each of which can be optionally substituted, to a C-terminal amino acid residue or a C-terminal peptide fragment, each of which can be optionally substituted, to form a compound having the formula
Y_{N}-Q-X_{C} I
where Y_{N} is H or an N-terminal amino acid residue or an N-terminal peptide fragment, each of which can be optionally substituted, Q is an acidic amino acid residue and X_{C} is a C-terminal amino acid residue or a C-terminal peptide fragment, each of which can be optionally substituted, comprising:
reacting a substrate component having the formula
Y_{N}-Q-OR II
where R is hydrogen, alkyl, haloalkyl, aryl, aralkyl, or a 5- or 6-membered heterocyclic ring having at least one heteroatom,
with an amino acid or peptide X_{C} in the presence of an endopeptidase in an aqueous solution or dispersion having a pH of from about 5 to about 10.5, to form the compound of formula I.

2. The process of claim 1 wherein said endopeptidase is of yeast, animal, vegetable, or microbial origin.

3. The process according to claim 1 or claim 2 wherein said endopeptidase cleaves specifically at the carboxyl side of an acidic amino acid residue.

4. The process according to any one of claims 1-3 wherein Q is an Asp or Glu residue.

5. The process according to any one of claims 1-4, wherein the substrate compound of formula II is Y_{N}-Asp(OH)-OR.

6. The process according to any one of claims 1-5, wherein R is ethyl.

7. The process according to any one of claims 1-6, wherein Y_{N} is desNH₂Tyr-D-Ala.

8. The process according to any one of claims 1-7, wherein X_{C} is SEQ ID NO:3-NH₂.

9. The process according to any one of claims 1-8 wherein the endopeptidase is V8 protease from S. aureus.

10. A process according to any one of claims 1-9 wherein a substrate component II having the formula
desNH₂-Tyr-D-Ala-Asp(OH)-OEt
is reacted with a compound X_{C} of formula 
Ala Ile Phe Thr Asn Ser Tyr Arg Lys Val Leu Ala Gln Leu Ser Ala
1               5                   10                  15
Arg Lys Leu Leu Gln Asp Ile Met Ser Arg-NH2
20 25
 (= SEQ ID No: 3-NH₂) to form a product of formula I having the sequence
